# EUROPEAN PATENT SPECIFICATION

(11) **EP 2 994 148 B1**
(45) Date of publication and mention of the grant of the patent: **01.02.2017**
(21) Application number: 14723864.6
(22) Date of filing: 09.05.2014
(51) Int. Cl.: A61K 35/74, A61K 31/436, A61P 35/00

(54) **CANCER THERAPY**
KREBSTHERAPIE
THÉRAPIE DU CANCER

(30) Priority: 09.05.2013 GB 201308325
(43) Date of publication of application: 16.03.2016
(73) Proprietor: Immodulon Therapeutics, London Wall London EC2M 5QQ (GB)
(72) Inventor: HAGEMANN, Thorsten, Whitechapel London E1 2EF (GB)
(74) Representative: Gill Jennings & Every LLP
(86) International application number: PCT/GB2014/051421
(87) International publication number: WO 2014/181121

(56) References cited:
- WO-A1-2013/079980
- WO-A2-2008/110491
- US-A1- 2013 209 517
- STEBBING J ET AL: "An intra-patient placebo-controlled phase I trial to evaluate the safety and tolerability of intradermal IMM-101 in melanoma", ANNALS OF ONCOLOGY, OXFORD UNIVERSITY PRESS, GB, vol. 23, no. 5, 1 May 2012 (2012-05-01), pages 1314-1319, XP008159930, ISSN: 1569-8041, DOI: 10.1093/ANNONC/MDR363 [retrieved on 2011-09-19]
- O'BRIEN M E R ET AL: "A RANDOMIZED PHASE II STUDY OF SRL172 (MYCOBACTERIUM VACCAE) COMBINED WITH CHEMOTHERAPY IN PATIENTS WITH ADVANCED INOPERABLE NON-SMALL-CELL LUNG CANCER AND MESOTHELIOMA", BRITISH JOURNAL OF CANCER, NATURE PUBLISHING GROUP, GB, vol. 83, no. 7, 1 October 2000 (2000-10-01), pages 853-857, XP001095848, ISSN: 0007-0920, DOI: 10.1054/BJOC.2000.1401

## Description

### FIELD OF THE INVENTION

The present invention relates to the field of cancer therapy. In particular, the present invention relates to a method of preventing, treating or inhibiting the development of tumours or metastases in a subject and to an immunomodulator for use in such therapy, in combination with an mTOR inhibitor.

### BACKGROUND OF THE INVENTION

In recent years there has been a growing realization that immune responses play a central role in cancer biology by eliminating many tumours at a very early stage and keeping those that avoid total elimination in a state of equilibrium, sometimes for many years (Dunn et al, Annu Rev Immunol 2004; 22:329-360). The eventual escape from this equilibrium phase with clinical manifestation of the disease is associated with dysregulated immune responses, manifesting, for example, as chronic inflammation or immunosuppression. The strong and increasing evidence that the immune system is critically involved in the development, structural nature and progression of cancer has led to renewed interest in immunotherapeutic strategies for treatment of this class of diseases. To date, most attempts to develop such strategies have been based on the use of antigens derived from the patient's own tumour or from tumour cell lines and the transfer of *ex-vivo* expanded populations of tumour antigen-specific cytotoxic cells and antigen-presenting cells.

Cancer has been associated with inflammation since 1863, when Rudolf Virchow discovered leucocytes in neoplastic tissues and so made the first connection between inflammation and cancer (Balkwill et al, Lancet 2001; 357:539-545). Since then, chronic inflammation has been deemed to be a risk factor for cancer. These reports demonstrate that an inflammatory environment predisposes to tumour development and is consistent with that observed at tumour sites. However, the relationship of cancer with inflammation is not limited to the onset of the disease due to chronic inflammation. Schwartsburd (Cancer Met Rev 2003; 22:95-102) proposed that chronic inflammation occurs due to tumour environment stress and that this generates a shield from the immune system. It has been recently demonstrated that the tumour microenvironment resembles an inflammation site, with significant support for tumour progression, through chemokines, cytokines, lymphocytes and macrophages which contribute to both the neovascularisation and vasal dilation for increased blood flow, the immunosuppression associated with the malignant disease, and tumour metastasis. Furthermore, this inflammation-site tumour-generated microenvironment, apart from its significant role in protection from the immune system and promotion of cancer progression, has an adverse effect on the success of current cancer treatments.

Moreover, metastatic cancer cells leave the tumour as microcolonies, containing lymphocytes and platelets as well as tumour cells. Inflammation continues to play a role at metastatic sites by creating a cytokine milieu conducive to tumour growth.

Immune homeostasis consists of a tightly regulated interplay of pro- and anti-inflammatory signals. For example, loss of the anti-inflammatory signals leads to chronic inflammation and proliferative signalling. Interestingly, cytokines that both promote and suppress proliferation of the tumour cells are produced at the tumour site. As in the case of cancer initiation, it is the imbalance between the effects of these various processes that results in tumour promotion.

It is believed that, to treat cancer, the most effective type of immune response is of a Type 1, which favours the induction of CD4+ Th1 cellular responses, and of CD8+ CTL responses. In the context of cancer immunotherapy, immunostimulants can be used, which promote the development of Th1 responses. For example, BCG (Bacillus Calmette-Guerin) an attenuated strain of *Mycobacterium. bovis* developed as a vaccine against *M. tuberculosis* infection is also used for treatment of various other conditions, such as bladder carcinoma and cutaneous melanoma. Intravesical instillation of BCG for superficial transitional cell carcinoma of the bladder is currently considered a first-line treatment for this disease. Although serious complications with intravesical BCG are uncommon, these can occur in individuals and can range from local symptoms to hepatitis, pneumonitis, sepsis, and death. SRL-172 is a heat-killed preparation of *M. vaccae,* a member of the same genus as BCG but with additional immunological properties, as it induces both immunoregulation of Th2 responses and Type 1 enhancing effects.

To date, a major barrier to attempts to develop effective immunotherapy for cancer has been an inability to break immunosuppression at the cancer site and restore normal networks of immune reactivity. The physiological approach of immunotherapy is to normalize the immune reactivity so that the endogenous tumour antigens would be recognized and effective cytolytic responses would be developed against cells bearing these antigens.

Anti-cancer immune responses accompanying the action of chemo- and radiotherapy have been reviewed in detail and show that such responses are critical to therapeutic success by eliminating residual cancer cells and maintaining micrometastases in a state of dormancy (Zitvogel et al, J Clin Invest 2008; 118:1991-2001). However, this reference makes it clear that there is no simple immunotherapeutic strategy available for consistently enhancing such immune responses.

Efforts have been made in the art to provide combined ablative and chemotherapies for the treatment of tumours. WO2000064476 and US20050187207 disclose the use of an immunoadjuvant in combination with photodynamic therapy for the treatment of metastatic tumours. These documents disclose that the immunoadjuvant comprises mycobacterial cell wall skeletons and de-3-O-acylated lipid A and is administered by injection into the tumour. Castano et al (Nat Rev Cancers 2006; 6:535), Korbelik et al (J Photochem Photobiol 1998; 44:151) and Korbelik et al (J Photochem Photobiol 2001; 73:403) also disclose the treatment of tumours using a combination of photodynamic therapy and the administration of mycobacterial cell wall extract as an immunoadjuvant. mycobacterial cell walls contain compounds such as trehalose dimycolate and muramyl dipeptide which are known immunostimulators. The mycobacterial cell wall extracts used in the prior art combination therapies also elicit pro-inflammatory cytokines, reactive nitrogen species and recruit leukocytes which are associated with pathology including weight loss due to TNF-α mediated cachexia, with associated lipidemia, hypoglycaemia and peritonitis with ischemic and hemorrhagic lesions in the GI tract. The prior art combination therapies may therefore exacerbate the inflammatory response and have severe side effects.

EP2085466 discloses the concept of administering attenuated live microorganisms together with a cancer drug, e.g. rapamycin. The attenuated microorganisms must retain the ability to infect macrophages, so as to induce apoptosis in macrophages.

WO2006/109300 discloses the use of mycobacterial components to treat donor cells prior to or after isolation of the donor cells for re-administration to a patient. The intention is to treat donor cells to lessen the effect of graft-versus-host disease.

An aim of the present invention is to solve the problems associated with the combination therapies for tumours observed in the prior art and, specifically, to provide a treatment for secondary cancers formed by metastasis of a primary cancer away from the site of the primary cancer.

### SUMMARY OF THE INVENTION

The present invention provides an effective method for treating and/or preventing cancer by employing an mTOR inhibitor which acts synergistically with a whole cell *Mycobacterium.* The present invention therefore provides a combination therapy of an mTOR inhibitor with a specific type of immunotherapy. The inventors have found that the combination of both therapies is synergistic beyond simple additive effects of each therapy used individually.

In a first aspect of the invention, there is a whole cell *Mycobacterium* for use in the treatment of neoplastic disease in combination with an mTOR inhibitor, wherein the *Mycobacterium* is a non-pathogenic heat-killed *Mycobacterium.*

In a second aspect of the invention is a method of preventing, treating, reducing, inhibiting and/or controlling a primary neoplasia, tumour or cancer, in a subject, wherein said method comprises simultaneously, separately or sequentially administering to the subject, a therapeutically effective amount of (i) an mTOR inhibitor, and (ii) a whole cell *Mycobacterium.*

In a third aspect of the invention there is a method of preventing, treating, reducing, inhibiting and/or controlling the formation or establishment of metastasis of a primary neoplasia, tumour or cancer at one or more sites distinct from a primary neoplasia, tumour or cancer, wherein said method comprises simultaneously, separately or sequentially administering to the subject, a therapeutically effective amount of (i) an mTOR inhibitor, and (ii) a whole cell *Mycobacterium,* wherein the *Mycobacterium* is a non-pathogenic heat-killed *Mycobacterium*.

The present invention therefore provides a combination therapy of an mTOR inhibitor together with a specific type of immunotherapy. The inventors have found that the combination of both therapies is synergistic beyond simple additive effects of each therapy used individually.

### DESCRIPTION OF THE DRAWINGS

The invention is described with reference to the following drawing, in which:

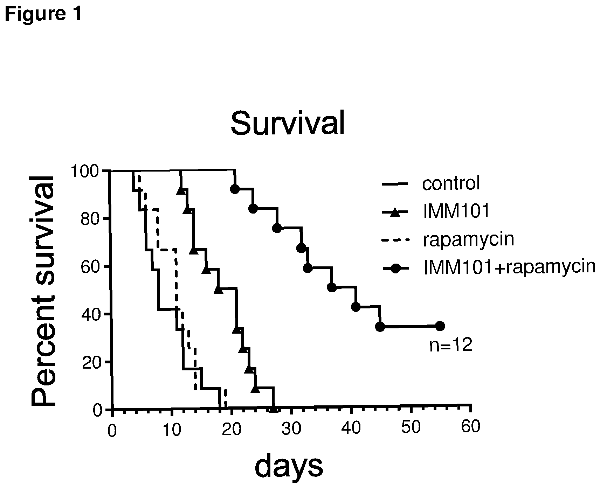

Figure 1 shows the effect of IMM-101 with or without co-administration of rapamycin, on survival in a spontaneous pancreatic cancer model in mice (KC mice) following orthotopic injection of KPC cells.

### DETAILED DESCRIPTION OF THE INVENTION

The terms "tumour", "cancer", "neoplasia" and "neoplastic disease" are used interchangeably and refer to a cell or population of cells whose growth, proliferation or survival is greater than the growth, proliferation or survival of a normal counterpart cell, e.g. a cell proliferative or differentiative disorders. Typically, the growth is uncontrolled. The term "malignancy" refers to invasion of nearby tissue. The term "metastasis" refers to spread or dissemination of a tumour, cancer or neoplasia to other sites, locations or regions within the subject, in which the sites, locations or regions are distinct from the primary tumour or cancer.

The term "mTOR" refers to the serine-threonine kinase Mammalian Target of Rapamycin. There are currently two recognized multi-molecular signaling forms of mTOR, mTOR complex1 (mTORC1) and mTOR complex 2 (mTORC2).

The term "inhibitor" has its conventional meaning as used in the art, that is namely, an entity which is able to bind to and decrease the activity of an enzyme. Preferably, the mTOR inhibitor is able to bind to and prevent or substantially prevent the formation of mTORC1 from its constituent components, so as to elicit a reduction in the activity of mTORC1; the mTOR inhibitor may also be able to bind to mTORC2 with likewise effects. More preferably, the mTOR inhibitor is selected from; sirolimus, everolimus, ridaforolimus, temsirolimus or metformin, or a pharmaceutically acceptable salt, solvate, hydrate, stereoisomer, clathrate, prodrug, analogue or derivative variant thereof thereof and/or combinations thereof. Most preferably, the mTOR inhibitor is sirolimus, or a derivative thereof as listed above.

As used herein, the terms "patient" and/or "subject" can be used interchangeably.

"Simultaneous" administration, as defined herein, includes the administration of the *Mycobacterium* and mTOR inhibitor within about 2 hours or about 1 hour or less of each other, even more preferably at the same time.

"Separate" administration, as defined herein, includes the administration of the *Mycobacterium* and mTOR inhibitor more than about 12 hours, or about 8 hours, or about 6 hours or about 4 hours or about 2 hours apart.

"Sequential" administration, as defined herein, includes the administration of the *Mycobacterium* and mTOR inhibitor each in multiple aliquots and/or doses and/or on separate occasions. Optionally, the *Mycobacterium* is administered before and continued to be administered to the subject after administration of the mTOR inhibitor. Alternatively, the *Mycobacterium* is continued to be applied to the subject after treatment for regression of the tumour.

The mammalian target of rapamycin (mTOR) pathway is a crucial regulator of cell growth and proliferation and research into this area has revealed that mTOR dysregulation has a key role to play in various cancers. mTOR appears to play a central role in signalling caused by nutrients and mitogens such as growth factors to regulate translation. The understanding of the science behind mTOR's role as a regulator of many cell processes and its potential as a therapeutic target has opened up treatment possibilities in several types of cancer. mTOR is a 290 kDa serine-threonine kinase that regulates both cell growth and cell cycle progression through its ability to integrate signals from nutrient and growth factor stimuli. mTOR, a member of the phosphatidylinositol 3-kinase (PI3K)-kinase-related kinase (PIKK) superfamily, is composed of 2549 amino acids that are grouped into highly conserved domains. The mTOR is an intracellular kinase that controls the production of proteins through effects on the machinery of mRNA translation. These proteins include important components of several processes critical to cell metabolism, cell growth, cell division, and responses to cellular stresses such as hypoxia or DNA damage.

mTOR senses the growth conditions within the cellular environment and helps the cells respond to changes in this environment. An active mTOR coordinates a response in cell growth directly through its effects on cell cycle regulators and indirectly by sustaining nutrient supply into the cell through the production of nutrient transporters and into the cell's environment through the promotion of angiogenesis. The activation of mTOR signifies a decision point that takes into account the availability of the basic materials required for cell growth (e.g., amino acids, glucose, energy) and the growth-regulating signals from other cells and tissues (e.g., hormones, growth factors) while monitoring conditions of cellular stress (e.g., hypoxia, DNA damage, heat shock, external pH, osmotic stress, oxidative stress). In this manner, the cell is protected from signals outside the cell to grow and proliferate originating when the supply of nutrients and energy inside the cell are not sufficient to support the effort.

Upstream in the growth-promoting pathways are critical molecules that converge on mTOR, which are often deregulated in some manner in cancer. Several mutations found in cancer produce inappropriate signals that activate the mTOR switch, driving the growth and proliferation of the cancer cell.

mTOR senses the availability of nutrients [e.g., adenosine triphosphate (ATP), glucose, amino acids, cholesterol, and iron] and consolidates this information with growth factor signalling through the PI3K/Akt/tuberous sclerosis complex (TSC) pathway. An activated mTOR modulates the rate of protein synthesis for select mRNAs by phosphorylating the translational protein S6 kinase (S6K) and 4E-binding protein 1 (4E-BP1). mTOR activation increases downstream effectors that stimulate cell growth and angiogenesis and regulate cellular metabolism.

A primary way that mTOR exerts its regulatory effects on cell proliferation is by controlling the production of cyclin D1. Cyclins are proteins that regulate the activity of enzymes called cyclin-dependent kinases (CDKs) through the critical G1-S restriction point of the cell cycle, which in turn regulate the passage of cells. Recently, cyclin D1 has been shown to play a role in gene transcription, cell metabolism, and cell migration.

Cyclin D1 overexpression had been associated with a number of cancers including breast cancer, colon cancer, prostate cancer, lymphoma, and melanoma.

Mutations in tumour sclerosis complex (TSC1 or TSC2) can also lead to overactivation of mTOR. This overactivation causes unregulated cell proliferation and multisystem tumours in patients with TSC. Increased mTOR activation, as evidenced by hyperphosphorylation of downstream signalling proteins, has been observed in TSC related lesions.

mTOR plays a key role in angiogenesis, i.e., the formation of new blood vessels to provide oxygen and nutrients to growing and dividing cells. mTOR increases the translation of hypoxia-inducible factor 1 (HIF-1)/hypoxia-inducible factor 2 (HIF- 2). The HIF transcription factors drive the expression of hypoxic stress response genes, including angiogenic growth factors such as vascular endothelial growth factor (VEGF), platelet-derived growth factor β (PDGF-β), and transforming growth factor-α (TGF-α)

Increased levels of HIF-1α and HIF-1β have been shown to correlate with increased mortality in a number of tumour types, including cervical cancer, breast cancer, non-small-cell lung cancer, ovarian cancer, head and neck cancer, and gastrointestinal stromal tumours. In addition, loss of the von Hippel-Lindau (VHL) protein, which also results in increased levels of HIF-1α, is a primary cause of many cases of renal cell carcinoma (RCC), and has been implicated in pancreatic cancer and neuroendocrine tumours (NETs) as well.

Bioenergetic research has shown that mTOR plays a key role in regulating cell metabolism. mTOR increases the surface expression of nutrient transporter proteins. An increase in these proteins results in greater uptake of amino acids and other nutrients by the cell, leading to adequate nutrient support for abnormal cell growth and survival. Additionally, abnormally activated mTOR may give cancer cells a competitive growth advantage by increasing production of the core enzymes necessary for glycolysis, which enables cancer cells to survive and grow even under hypoxic conditions.

There are several lines of evidence that mTOR activity plays a role in cell survival. Majority of this research has revealed that mTOR inhibition increases sensitivity to cell death pathways; however, there is also emerging evidence that mTOR activation may play a role in promoting cell survival through the activation of anti-apoptotic proteins that contribute to tumour progression.

Overactivation of mTOR due to dysregulation of upstream pathways, leading to abnormal activities in cell progression, angiogenesis, cell metabolism and apoptosis has been implicated in various cancer types.

### RENAL CELL CARCINOMA

mTOR controls production of HIF-1α, an important protein in RCC, where its unregulated activity is causally related to disease pathogenesis. mTOR regulates the production of several angiogenic growth factors in RCC. mTOR may control the ability of neovascular cells to respond to growth factors. mTOR controls cell growth and cell division in RCC and in cells of the tumour microvasculature, and is often dysregulated in renal cancer by signalling defects upstream of mTOR in the PI3K/Akt/mTOR pathway. mTOR regulates nutrient uptake and cell metabolism and contributes to the characteristic metabolic changes in RCC.

### NEUROENDOCRINE TUMOUR (NET)

Several important molecular changes in NETs involve the mTOR pathway. Increased growth factor signalling, namely, epidermal growth factor (EGF) and insulin-like growth factor (IGF) signalling upstream of mTOR, has been observed frequently in NETs. Also, insulin secretion is believed to be involved in the autocrine activation of mTOR in pancreatic beta cell tumours. mTOR is activated by many gene mutations associated with NETs (germline deletion of the VHL gene). mTOR directs the supply of nutrients to cancer cells by regulating angiogenesis. NETs are highly vascular. VEGF expression has been observed in 80-86% of gastrointestinal carcinoid and pancreatic islet cell tumours.

### GASTRIC CANCER

mTOR is activated in 60-80% of gastric adenocarcinomas and is expressed in early-stage and advanced-stage disease, in both diffuse and intestinal subtypes, and in tumour cells that invade lymphatic channels. The mTOR pathway is activated by multiple growth factor receptors, namely, epidermal growth factor receptor (EGFR), Human Epidermal growth factor Receptor 2 (HER2), insulin-like growth factor type 1 receptor (IGF-1R), that are overexpressed in many gastric tumours. mTOR regulates production of angiogenic factors (VEGF/VEGFR) that promote new vessel formation and predict poor outcome in patients with gastric cancer. mTOR regulates nutrient uptake and cell metabolism and contributes to the characteristic metabolic changes in cancer. HIF-1α is expressed in most gastric cancers, and HIF-1α expression at the invading tumour edge is associated with advanced-stage disease, lymph node metastases, and poor survival.

### BREAST CANCER

mTOR signalling is critical in the pathogenesis of breast cancer. mTOR signalling may be related to estrogen receptor (ER) activation and adaptive estrogen hypersensitivity. mTOR pathway signalling is increased in HER2+ tumour cells resistant to endocrine therapy. mTOR activation predicts a worse clinical outcome for patients treated with endocrine therapy. mTOR controls the supply of nutrients to cancer cells by regulating nutrient uptake, cell metabolism, and angiogenesis.

### HEPATOCELLULAR CARCINOMA (HCC)

mTOR-dependent signalling is active in 25-45% of HCC. Activation correlates with shorter overall survival; mTOR activation is an independent predictor of recurrence after surgery. mTOR regulates production of angiogenic factors. High VEGF levels have been associated with tumour cell proliferation, poor encapsulation of the tumour nodules, venous invasion, higher grade, and a poor prognosis following resection. mTOR activation through PI3K/Akt pathway is associated with increased expression of growth factors such as EGF, TGF-α, IGF, and hepatocyte growth factor (HGF) that promote HCC cell proliferation and survival.

### LYMPHOMA

Approximately 85% of Non-Hodgkin lymphoma (NHL) arise from cells of B-cell lineage. mTOR signalling is activated in Hodgkin lymphoma cell lines and primary tumours. Cyclin D1 overexpression is a characteristic feature of mantle cell lymphomas. PI3K and Akt overexpression is frequently observed in several B-cell lymphomas.

In one aspect of the present invention the whole cell *Mycobacterium* is heat-killed. Examples of mycobacterial species for use in the present invention include *M. vaccae, M. thermoresistibile, M. flavescens, M. duvalii, M. phlei, M. obuense, M. parafortuitum, M. sphagni, M. aichiense, M. rhodesiae, M. neoaurum, M. chubuense, M. tokaiense, M. komossense, M. aurum, M. indicus pranii, M. tuberculosis, M. microti; M. africanum; M. kansasii, M. marinum; M. simiae; M. gastri; M. nonchromogenicum; M. terrae; M. triviale; M. gordonae; M. scrofulaceum; M. paraffinicum; M. intracellulare; M. avium; M. xenopi; M. ulcerans; M. diernhoferi, M. smegmatis; M. thamnopheos; M. flavescens; M. fortuitum; M. peregrinum; M. chelonei; M. paratuberculosis; M. leprae; M. lepraemurium* and combinations thereof.

The heat-killed *Mycobacterium* is non-pathogenic. The non-pathogenic heat-killed *Mycobacterium* is preferably selected from *M. vaccae, M. obuense, M. parafortuitum, M. aurum, M. indicus pranii, M. phlei* and combinations thereof. More preferably the non-pathogenic heat-killed *Mycobacterium* is a rough variant. The amount of *Mycobacterium* administered to the subject is sufficient to elicit a protective immune response in the subject such that the subject's immune system is able to mount an effective immune response to the cancer. In certain embodiments of the invention, it is preferable that a particular dosage of *Mycobacterium* and/or dosing schedule be administered to a subject. Thus, in certain embodiments of the invention, there is provided a containment means comprising the effective amount of heat-killed *Mycobacterium* for use in the present invention, which typically may be from 10³ to 10¹¹ organisms, preferably from 10⁴ to 10¹⁰ organisms, more preferably from 10⁶ to 10¹⁰ organisms, and even more preferably from 10⁶ to 10⁹ organisms. The effective amount of heat-killed *Mycobacterium* for use in the present invention may be from 10³ to 10¹¹ organisms, preferably from 10⁴ to 10¹⁰ organisms, more preferably from 10⁶ to 10¹⁰ organisms, and even more preferably from 10⁶ to 10⁹ organisms. Most preferably the amount of heat-killed *Mycobacterium* for use in the present invention is from 10⁷ to 10⁹ cells or organisms. Typically, the composition according to the present invention may be administered at a dose of from 10⁸ to 10⁹ cells for human and animal use. Alternatively the dose is between about 0.01 mg to 1 mg, or between about 0.1mg to 1mg by weight of organisms, presented as either a suspension or dry preparation.
*M. vaccae* and *M. obuense* are particularly preferred.

*M. vaccae* and *M. obuense* induce a complex immune response in the host. Treatment with these preparations will stimulate innate and type-1 immunity (Th1), including macrophage activation and cytotoxic cell activity. They also independently down-regulate inappropriate Th2 responses via immunoregulatory mechanisms. This restores the healthy balance of the immune system.

The present invention may be used to treat a neoplastic disease. As used herein, "treatment" encompasses the prevention, reduction, control and/or inhibition of a neoplastic disease. Such diseases include a sarcoma, carcinoma, adenocarcinoma, melanoma, myeloma, blastoma, glioma, lymphoma or leukemia. Exemplary cancers include, carcinoma, sarcoma, adenocarcinoma, melanoma, neural (blastoma, glioma), mesothelioma and reticuloendothelial, lymphatic or haematopoietic neoplastic disorders (e.g., myeloma, lymphoma or leukemia). In particular aspects, a neoplasm, tumour or cancer includes a lung adenocarcinoma, lung carcinoma, diffuse or interstitial gastric carcinoma, colon adenocarcinoma, prostate adenocarcinoma, esophagus carcinoma, breast carcinoma, pancreas adenocarcinoma, ovarian adenocarcinoma, adenocarcinoma of the adrenal gland, adenocarcinoma of the endometrium or uterine adenocarcinoma.

Neoplasia, tumours and cancers include benign, malignant, metastatic and non-metastatic types, and include any stage (I, II, III, IV or V) or grade (G1, G2, G3, etc.) of neoplasia, tumour, or cancer, or a neoplasia, tumour, cancer or metastasis that is progressing, worsening, stabilized or in remission. Cancers that may be treated according to the invention include but are not limited to cells or neoplasms of the bladder, blood, bone, bone marrow, brain, breast, colon, esophagus, gastrointestines, gum, head, kidney, liver, lung, nasopharynx, neck, ovary, prostate, skin, stomach, testis, tongue, or uterus. In addition, the cancer may specifically be of the following histological type, though it is not limited to the following: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumour, malignant; bronchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; Paget's disease, mammary; acinar cell carcinoma; adenosquamous carcinoma; adenocarcinoma with squamous metaplasia; thymoma, malignant; ovarian stromal tumour, malignant; thecoma, malignant; granulosa cell tumour, malignant; androblastoma, malignant; Sertoli cell carcinoma; Leydig cell tumour, malignant; lipid cell tumour, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumour; Mullerian mixed tumour; nephroblastoma; hepatoblastoma; carcinosarcoma; mesenchymoma, malignant; Brenner tumour, malignant; phyllodes tumour, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; Kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumour of bone; Ewing's sarcoma; odontogenic tumour, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumour; meningioma, malignant; neurofibrosarcoma; neurilemmoma, malignant; granular cell tumour, malignant; malignant lymphoma; Hodgkin's disease; Hodgkin's; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia. Preferably, the neoplastic disease may be tumours associated with a cancer selected from prostate cancer, liver cancer, renal cancer, lung cancer, breast cancer, colorectal cancer, pancreatic cancer, brain cancer, hepatocellular cancer, lymphoma, leukaemia, gastric cancer, cervical cancer, ovarian cancer, thyroid cancer, melanoma, head and neck cancer, skin cancer and soft tissue sarcoma and/or other forms of carcinoma. The tumour may be metastatic or a malignant tumour.

More preferably, the neoplastic disease to be treated is pancreatic cancer, breast cancer, prostate cancer and skin cancer. Most preferably, the neoplastic disease to be treated is pancreatic cancer.

In an embodiment of the invention, the mTOR inhibitor, in combination therapy with a *Mycobacterium,* is used to reduce or inhibit metastasis of a primary tumour or cancer to other sites, or the formation or establishment of metastatic tumours or cancers at other sites distal from the primary tumour or cancer thereby inhibiting or reducing tumour or cancer relapse or tumour or cancer progression.

In further embodiments, methods of the invention include, among other things, 1) reducing or inhibiting growth, proliferation, mobility or invasiveness of tumour or cancer cells that potentially do develop metastases, 2) reducing or inhibiting formation or establishment of metastases arising from a primary tumour or cancer to one or more other sites, locations or regions distinct from the primary tumour or cancer; 3) reducing or inhibiting growth or proliferation of a metastasis at one or more other sites, locations or regions distinct from the primary tumour or cancer after a metastasis has formed or has been established, and 4) reducing or inhibiting formation or establishment of additional metastasis after the metastasis has been formed or established.

In an embodiment of the invention, administration of the mTOR inhibitor in combination therapy with a *Mycobacterium,* provides a detectable or measurable improvement in a condition of a given subject, such as alleviating or ameliorating one or more adverse (physical) symptoms or consequences associated with the presence of a cell proliferative or cellular hyperproliferative disorder, neoplasia, tumour or cancer, or metastasis, i.e., a therapeutic benefit or a beneficial effect.

A therapeutic benefit or beneficial effect is any objective or subjective, transient, temporary, or long-term improvement in the condition or pathology, or a reduction in onset, severity, duration or frequency of an adverse symptom associated with or caused by cell proliferation or a cellular hyperproliferative disorder such as a neoplasia, tumour or cancer, or metastasis. A satisfactory clinical endpoint of a treatment method in accordance with the invention is achieved, for example, when there is an incremental or a partial reduction in severity, duration or frequency of one or more associated pathologies, adverse symptoms or complications, or inhibition or reversal of one or more of the physiological, biochemical or cellular manifestations or characteristics of cell proliferation or a cellular hyperproliferative disorder such as a neoplasia, tumour or cancer, or metastasis. A therapeutic benefit or improvement therefore may be reduction in adverse symptoms or complications associated with or caused by cell proliferation or the cellular hyperproliferative disorder such as a neoplasia, tumour or cancer, or metastasis. However, a therapeutic benefit or improvement need not be a cure or complete destruction of all target proliferating cells (e.g., neoplasia, tumour or cancer, or metastasis). For example, stabilization of the tumour or cancer mass, size or cell numbers by inhibiting progression or worsening of the tumour or cancer, can reduce mortality and prolong lifespan even if only for a few days, weeks or months, even though a portion or the bulk of the tumour or cancer mass, size or cells remain.

Specific non-limiting examples of therapeutic benefit include a reduction in neoplasia, tumour or cancer, or metastasis volume (size or cell mass) or numbers of cells, inhibiting or preventing an increase in neoplasia, tumour or cancer volume (e.g., stabilizing), slowing or inhibiting neoplasia, tumour or cancer progression, worsening or metastasis, or inhibiting neoplasia, tumour or cancer proliferation, growth or metastasis.

An invention method may not take effect immediately. For example, treatment may be followed by a delayed effect on tumour volume and/or an initial increase in the neoplasia, tumour or cancer cell numbers or mass, but over time eventual stabilization or reduction in tumour cell mass, size or numbers of cells in a given subject may subsequently occur.

Additional adverse symptoms and complications associated with neoplasia, tumour, cancer and metastasis that can be inhibited, reduced, decreased, delayed or prevented include, for example, nausea, lack of appetite, lethargy, pain and discomfort. Thus, a partial or complete decrease or reduction in the severity, duration or frequency of an adverse symptom or complication associated with or caused by a cellular hyperproliferative disorder, an improvement in the subjects well-being, such as increased energy, appetite, psychological well-being, are all particular non-limiting examples of therapeutic benefit.

A therapeutic benefit or improvement therefore can also include a subjective improvement in the quality of life of a treated subject.

In an additional embodiment, a method prolongs or extends lifespan (survival) of the subject. In a further embodiment, a method improves the quality of life of the subject.

The terms "effective amount" or "therapeutically effective amount" refer to a sufficient amount of an agent to provide the desired biological, therapeutic, and/or prophylactic result. That result can be reduction, amelioration, palliation, lessening, delaying, and/or alleviation of one or more of the signs, symptoms, or causes of a disease, or any other desired alteration of a biological system. In reference to cancer, an effective amount comprises an amount sufficient to cause a tumour to shrink and/or to decrease the growth rate of the tumour (such as to suppress tumour growth) or to prevent or delay other unwanted cell proliferation. In some embodiments, an effective amount is an amount sufficient to delay development. In some embodiments, an effective amount is an amount sufficient to prevent or delay recurrence. An effective amount can be administered in one or more administrations. The effective amount of the drug or composition may: (i) reduce the number of cancer cells; (ii) reduce tumour size; (iii) inhibit, retard, slow to some extent and preferably stop cancer cell infiltration into peripheral organs; (iv) inhibit (i.e., slow to some extent and preferably stop) tumour metastasis; (v) inhibit tumour growth; (vi) prevent or delay occurrence and/or recurrence of tumour; and/or (vii) relieve to some extent one or more of the symptoms associated with the cancer.

In a most preferred embodiment, administration of the mTOR inhibitor, in combination therapy with a *Mycobacterium,* results in a clinically relevant improvement in one or more markers of disease status and progression selected from one or more of the following: (i) overall survival, (ii) progression-free survival, (iii) overall response rate, (iv) reduction in metastatic disease, (v) circulating levels of cancer-associated antigen, where relevant, (vi) nutritional status (weight, appetite, serum albumin), or (vii) pain control or analgesic use.

Pre-treatment with heat-killed whole cell *M. vaccae* and *M. obuense* gives rise to more complex immunity including not only the development of innate immunity and Type-1 immunity, but also immunoregulation which more efficiently restores appropriate immune functions.

The *Mycobacterium* according to the invention is administered in combination with an mTOR inhibitor.

In a preferred embodiment, the mTOR inhibitor may be selected from sirolimus, everolimus, ridaforolimus, temsirolimus or metformin or a pharmaceutically acceptable salt, solvate, hydrate, stereoisomer, clathrate, or prodrug, analogue or derivative variant of the foregoing, or combinations thereof.

In a most preferred embodiment, the mTOR inhibitor is rapamycin (sirolimus).

The term "combination" as used throughout the specification, is meant to encompass the administration of the mTOR inhibitor simultaneously, separately or sequentially with administration of the *Mycobacterium.* Accordingly, the mTOR inhibitor and the *Mycobacterium* may be present in the same or separate pharmaceutical formulations, and at the same time or at different times.

Thus, a *Mycobacterium* and the mTOR inhibitor may be provided as separate medicaments for administration at the same time or at different times.

Preferably, a *Mycobacterium* and mTOR inhibitor are provided as separate medicaments for administration at different times. When administered separately and at different times, either the *Mycobacterium* or mTOR inhibitor may be administered first; however, it is preferable to administer the *Mycobacterium* followed by mTOR inhibitor. In addition, both can be administered on the same day or at different days, and they can be administered using the same schedule or at different schedules during the treatment cycle.

The duration of each cycle of mTOR inhibitor may be between about 1 to about 3 days or up to about 52 weeks, or longer. Multiple cycles for each medicament can be given as needed. Thus, in an embodiment of the invention, a treatment cycle consists of the administration of the *Mycobacterium* daily, weekly, fortnightly or monthly simultaneously with mTOR inhibitor. Alternatively, the *Mycobacterium* is administered before and/or after the administration of the mTOR inhibitor.

Dose delays and/or dose reductions and schedule adjustments are performed as needed depending on individual patient tolerance to treatments.

Effective amounts of *Mycobacterium* may be administered in multiple (repeat) doses, for example two or more, three or more, four or more, five or more, ten or more, or twenty or more repeat doses, optionally at intervals of about two days or about 2 weeks, or about 4 weeks or about 8 weeks.

In another embodiment, the treatment regimen comprises administration of the *Mycobacterium* every 2 days or up to every two weeks for the first 3 doses followed by a rest of 4 weeks then every 2 weeks for the next 3 doses followed by every 4 weeks thereafter, with mTOR inhibitor administration beginning at least between 1 day and 14 days after first dose of said *Mycobacterium.*

In yet a further embodiment of the invention, the effective amount of the mTOR inhibitor, optionally between about 0.5 mg/day and about 40 mg/day, such as between about 1.5 mg/day and about 15 mg/day, optionally via the oral route, may be administered in multiple (repeat) daily doses, for example two or more, three or more, four or more, five or more, ten or more, or twenty or more repeat doses, before, concurrently with and/or after administration of the *Mycobacterium.* For example, both the *Mycobacterium* and mTOR inhibitor are administered and repeated on at least about 2, 4, 6, 8, 10, 12, 15, 20 or more occasions, optionally wherein the mTOR inhibitor is administered daily.

In a further embodiment of the invention, the effective amount of the mTOR inhibitor, optionally between about 0.5 mg/day and about 40 mg/day, such as between about 1.5 mg/day and about 20 mg/day, or between about 5 mg/day and 10 mg/day, optionally via the oral route and wherein the mTOR inhibitor is everolimus, may be administered in multiple (repeat) daily doses, for example two or more, three or more, four or more, five or more, ten or more, or twenty or more repeat doses, before, concurrently with and/or after administration of the *Mycobacterium.*

In a further embodiment of the invention, the effective amount of the mTOR inhibitor, optionally between about 0.5 mg/day and about 40 mg/day, such as between about 2 mg/day and about 6 mg/day, optionally via the oral route and wherein the mTOR inhibitor is rapamycin, may be administered in multiple (repeat) daily doses, for example two or more, three or more, four or more, five or more, ten or more, or twenty or more repeat doses, before, concurrently with and/or after administration of the *Mycobacterium.*

The *Mycobacterium* may be administered to the patient via the parenteral, oral, sublingual, nasal or pulmonary route. In a preferred embodiment, the *Mycobacterium* is administered via a parenteral route selected from subcutaneous, intradermal, subdermal, intraperitoneal, intravenous and intravesicular injection. More preferably, administration by the parenteral route does not comprise intratumoural injection of mycobacterial cell wall extract.

A dosage schedule according to the present invention may include administration of the *Mycobacterium* between about 0.25 hours and about 2 weeks prior to and on the day of said mTOR inhibitor administration, followed by further doses of said *Mycobacterium* between about every two days or about every 2 weeks or about 4 weeks later. Further doses of *Mycobacterium* may be administered at daily or weekly or fortnightly or monthly intervals such as over a period of about 8 weeks, or about 10 weeks and about 12 weeks, or longer. The *Mycobacterium* may continue to be administered for up to 12 months or more following the first dose given.

The subject whom is to undergo mTOR inhibitor therapy according to the present invention may do so simultaneously, separately or sequentially with administration of the *Mycobacterium.* The *Mycobacterium* may be administered to the patient prior to administration of an mTOR inhibitor. More specifically, the *Mycobacterium* may be administered to the patient between about 4 weeks and about 1 day or less prior to the mTOR inhibitor administration. Alternatively, the *Mycobacterium* may be administered as one or more aliquots each containing an effective amount of the *Mycobacterium* which may be administered at one or more time intervals between 4 weeks and about 1 day or less prior to mTOR inhibitor administration and/or the *Mycobacterium* may be applied after administration of an mTOR inhibitor. In a further alternative, the *Mycobacterium* may be administered as one or more aliquots each containing an effective amount of the *Mycobacterium* which may be administered at one or more time intervals between 4 weeks and about 1 day or less after the mTOR inhibitor and/or the *Mycobacterium* may applied after administration of an mTOR inhibitor, and repeated on at least about 2, 4, 6, 8, 10, 12, 15, 20 or more occasions before and/or after administration of an mTOR inhibitor.

In a preferred embodiment of the invention there is a *Mycobacterium* for use in the treatment of neoplastic disease in combination with an mTOR inhibitor wherein said *Mycobacterium* is administered to the subject before, concurrently with and/or after the mTOR inhibitor is administered.

In a further preferred embodiment of the invention there is a *Mycobacterium* for use in the treatment of neoplastic disease in combination with an mTOR inhibitor wherein is administered to the subject before, concurrently with and/or after said mTOR inhibitor is administered, wherein said *Mycobacterium* and/or mTOR inhibitor is to be administered in repeat doses.

In yet a further preferred embodiment of the invention is a method of preventing, treating, reducing, inhibiting and/or controlling a primary neoplasia, tumour or cancer, in a subject, wherein said method comprises simultaneously, separately or sequentially administering to the subject, a therapeutically effective amount of (i) an mTOR inhibitor, and (ii) a whole cell *Mycobacterium,* wherein said *Mycobacterium* is administered to the subject before, concurrently with and/or after said mTOR inhibitor is administered, optionally wherein said *Mycobacterium* and/or mTOR inhibitor is to be administered in repeat doses.

In another preferred embodiment of the invention is a method of preventing, treating, reducing, inhibiting and/or controlling the formation or establishment of metastasis of a primary neoplasia, tumour or cancer at one or more sites distinct from a primary neoplasia, tumour or cancer, wherein said method comprises simultaneously, separately or sequentially administering to the subject, a therapeutically effective amount of (i) an mTOR inhibitor, and (ii) a whole cell *Mycobacterium,* wherein said *Mycobacterium* is administered to the subject before, concurrently with and/or after said mTOR inhibitor is administered, optionally wherein said *Mycobacterium* and/or mTOR inhibitor is to be administered in repeat doses.

In one embodiment of the present invention, the *Mycobacterium* may be in the form of a medicament administered to the patient in a dosage form.

In a further embodiment of the invention, the effective amount of the *Mycobacterium* may be administered as a single dose. Alternatively, the effective amount of the *Mycobacterium* may be administered in multiple (repeat) doses, for example two or more, three or more, four or more, five or more, ten or more, or twenty or more repeat doses. For example, the *Mycobacterium* is administered between about 4 weeks and about 1 day or less prior to mTOR inhibitor administration. Administration may be presented in single or multiple doses.

In yet a further embodiment of the invention, the effective amount of the *Mycobacterium* may be administered in multiple (repeat) doses, for example two or more, three or more, four or more, five or more, ten or more, or twenty or more repeat doses concomitantly with administration of an mTOR inhibitor. For example, both the *Mycobacterium* and mTOR inhibitor is administered and repeated on at least about 2, 4, 6, 8, 10, 12, 15, 20 or more occasions, optionally wherein the mTOR inhibitor is administered daily. Administration of both the *Mycobacterium* and mTOR inhibitor may be presented in multiple doses.

In yet a further embodiment of the invention, the effective amount of the mTOR inhibitor, optionally between about between about 0.5 mg/day and about 40 mg/day, such as between about 1.5 mg/day and about 20 mg/day, or between about 5 mg/day and 10 mg/day, optionally via the oral route may be administered in multiple (repeat) daily doses, for example two or more, three or more, four or more, five or more, ten or more, or twenty or more repeat doses, before, concurrently with and/or after administration of the *Mycobacterium.* For example, both the *Mycobacterium* and mTOR inhibitor is administered and repeated on at least about 2, 4, 6, 8, 10, 12, 15, 20 or more occasions, optionally wherein the mTOR inhibitor is administered daily.

A container according to the invention in certain instances, may be a vial, an ampoule, a syringe, capsule, tablet or a tube. In some cases, the mycobacteria may be lyophilized and formulated for resuspension prior to administration.

However, in other cases, the mycobacteria are suspended in a volume of a pharmaceutically acceptable liquid. In some of the most preferred embodiments there is provided a container comprising a single unit dose of mycobacteria suspended in pharmaceutically acceptable carrier wherein the unit dose comprises about 1 x 10⁶ to about 1 x 10¹⁰ CFU of mycobacteria. In some very specific embodiments the liquid comprising suspended mycobacteria is provided in a volume of between about 0.1 ml and 10 ml, or between about 0.3 ml and 2 ml or between about 0.5 ml and 2 ml. It will further be understood that in certain instances a composition comprising mycobacteria in a containment means is frozen (i.e. maintained at less than about zero degrees Celsius). The foregoing compositions provide ideal units for immunotherapeutic applications described herein.

Embodiments discussed in the context of a methods and/or composition of the invention may be employed with respect to any other method or composition described herein. Thus, an embodiment pertaining to one method or composition may be applied to other methods and compositions of the invention as well.

In some cases non-pathogenic heat-killed mycobacteria is administered to specific sites on or in a subject. For example, the mycobacterial compositions according to the invention, such as those comprising *M. obuense* in particular, may be administered adjacent to tumours or adjacent to lymph nodes, such as those that drain tissue surrounding a tumour. Thus, in certain instances sites administration of mycobacterial composition may be near the posterior cervical, tonsillar, axillary, inguinal, anterior cervical, sub-mandibular, sub mental or superclavicular lymph nodes. Such sites of administration may be on the right side, on the left side, or on both sides of the body. In certain very specific embodiments, mycobacterial compositions are delivered close to the axillary, cervical and/or inguinal lymph nodes. For example, a dosage of the mycobacteria may distribute into tissues adjacent to the right and left axillary lymph node and the right and left inguinal lymph nodes.

In a very specific embodiment a dosage of mycobacteria is administered to a subject by intradermal injection wherein the dosage is distributed to the axillary and inguinal on both sides of the body and wherein there are two injections (i.e. two wheals) at each site.

In some further embodiments of the invention, methods of the invention involve the administration of 2, 3, 4, 5, 6, 7, 8, 9, 10 or more doses of mycobacteria separated by a period of one day or more. For example, such separate doses may be separated by a single day, or several days, one week, two weeks, one month or more. For example, methods according to the invention may comprise administering 1 to 5 doses of mycobacteria over a period of three weeks or more. In yet further embodiments, methods of the invention comprise administering 1 to 5, 1 to 4, 1 to 3, 1 to 2 or 2 doses of mycobacteria over a period of about one to about three weeks, or more. Each dose administered may be the same or different dosage relative to a previous or subsequent dose administration. For example, in certain cases, it is preferred that a dosage of mycobacteria is lower than any dosage that was previously administered. Thus, in some specific cases, a dose of non-pathogenic, heat-killed mycobacteria will be administered at about half of the dosage that was administered in any previous treatment. Such methods may be preferred in certain instances where the subject's immune response to the mycobacteria is greater during subsequent therapies. Thus in certain cases, the *Mycobacterium* may be administered a minimal number of times for example, in less than 10, 9, 8, 7, 6, 5, 4, 3 or fewer separate dosage administrations. In some cases the mycobacterial composition is administered twice. Alternatively, the *Mycobacterium* may be administered for the length of time the cancer or tumour(s) is present in a patient or until such time the cancer has regressed or stabilized. The *Mycobacterium* may also be continued to be administered to the patients once the cancer or tumour has regressed or stabilised.

Mycobacterial compositions according to the invention will comprise an effective amount of mycobacteria typically dispersed in a pharmaceutically acceptable carrier. The phrases "pharmaceutical or pharmacologically acceptable" refers to molecular entities and compositions that do not produce an adverse, allergic or other untoward reaction when administered to an animal, such as, for example, a human, as appropriate. The preparation of an pharmaceutical composition that contains mycobacteria will be known to those of skill in the art in light of the present disclosure, as exemplified by Remington's Pharmaceutical Sciences, 18th Ed. Mack Printing Company, 1990, Moreover, for animal (e.g., human) administration, it will be understood that preparations should meet sterility, pyrogenicity, general safety and purity standards. A specific example of a pharmacologically acceptable carrier as described herein is borate buffer or sterile saline solution (0.9% NaCl).

As used herein, "pharmaceutically acceptable carrier" includes any and all solvents, dispersion media, coatings, surfactants, antioxidants, preservatives {e.g., antibacterial agents, antifungal agents), isotonic agents, absorption delaying agents, salts, preservatives, drugs, drug stabilizers, gels, binders, excipients, disintegration agents, lubricants, sweetening agents, flavouring agents, dyes, such like materials and combinations thereof, as would be known to one of ordinary skill in the art (see, for example, Remington's Pharmaceutical Sciences, 18th Ed. Mack Printing Company, 1990, pp. 1289-1329).

In a preferred embodiment, the *Mycobacterium* is administered via a parenteral route selected from subcutaneous, intradermal, subdermal, intraperitoneal, intravenous and intravesicular injection. Intradermal injection enables delivery of an entire proportion of the mycobacterial composition to a layer of the dermis that is accessible to immune surveillance and thus capable of electing an anti-cancer immune response and promoting immune cell proliferation at local lymph nodes.

Though in highly preferred embodiments of the invention mycobacterial compositions are administered by direct intradermal injection, it is also contemplated that other methods of administration may be used in some case. Thus in certain instances heat-killed mycobacteria of the present invention can be administered by injection, infusion, continuous infusion, intravenously, intradermally, intraarterially, intraperitoneally, intralesionally, intravitreally, intravaginally, intrarectally, topically, intratumourally, intramuscularly, intraperitoneally, subcutaneously, subconjunctival, intravesicularlly, mucosally, intrapericardially, intraumbilically, intraocularally, orally, intracranially, intraarticularly, intraprostaticaly, intrapleurally, intratracheally, intranasally, topically, locally, inhalation (e.g. aerosol inhalation), via a catheter, via a lavage, or by other method or any combination of the forgoing as would be known to one of ordinary skill in the art (see, for example, Remington's Pharmaceutical Sciences, 18th Ed. Mack Printing Company, 1990). More preferably, administration by the parenteral route does not comprise intratumoural injection of mycobacterial cell wall extract.

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and immunology or related fields are intended to be within the scope of the following claims.

The invention is further described with reference to the following non-limiting Example.

### Example 1

This example describes a study investigating the administration of heat-killed whole cell *Mycobacterium obuense* (IMM-101) and the mTor inhibitor Rapamycin (Sirolimus) in a well-validated, clinically relevant genetic mouse model of pancreatic cancer (pancreatic ductal adenocarcinoma). Genetically-modified mice bearing mutations in Kras and Pdx-Cre (KC mice) were bred according to the method described by Hingorani et al. (Cancer Cell, 2003, 4:437-50); a proportion of these mice develop ductal lesions similar to human pancreatic intraepithelial neoplasias which may progress to invasive and metastatic adenocarcinoma. KC mice were injected with 10⁵ KPC cells bearing a mutation in Kras, p53 and Pdx-Cre (Hingorani et al. Cancer Cell, 2005, 7:469-48) orthotopically on day 100 after birth. Mice were treated as follows on day 114 (day 0 in Figure 1):
- 12 mice untreated (control);
- 12 mice treated with 2mg/kg Rapamycin, intraperitoneally, daily, for the length of the study;
- 12 mice treated with 0.1mg IMM-101/mouse, subcutaneously alternating injections in the scruff of the neck with ones at the base of the tail on alternating days over 5 day period with 2 day break for the length of the study;
- 12 mice treated with the combination of Rapamycin and IMM-101 at a dose and schedule described above for the two compounds used singly.

As expected, mice left untreated succumbed to disease within 20 days. Treatment with an mTor inhibitor failed to have a significant effect on survival, with mice treated with Rapamycin living just as long as control animals. However, most surprisingly, the combination of an mTOR inhibitor (Rapamycin) and mycobacteria (IMM-101) *significantly* extended survival of such treated mice compared to control mice and most importantly compared to mice treated with the compounds used singly (see Figure 1). It appears that the combination of the effects of mTor inhibition with those mediated by a *Mycobacterium* acts in synergy in an unexpected manner, which cannot be explained by their effects when used singly.

### References

Apetoh L, Ghiringhelli F, Tesniere A, et al. Cancer is not just a disease of a tissue: it is a host disease. How to reactivate host defense against tumours using conventional therapies of cancer? Ann Endocrinol (Paris) 2008a;69:151-152.
Apetoh L, Tesniere A, Ghiringhelli F, et al. Molecular interactions between dying tumour cells and the innate immune system determine the efficacy of conventional anticancer therapies. Cancer Res 2008b;68:4026-30.
Dunn GP, Old LJ, Schreiber RD. The three Es of cancer immunoediting. Annu Rev Immunol 2004; 22:329-360.
Hingorani SR, Petricoin III EF, Maitra A, et al. Preinvasive and invasive ductal pancreatic cancer and its early detection in the mouse. Cancer Cell 2003; 4:437-450.
Hingorani SR, Wang L, Multani AS et al. Trp53R172H and KrasG12D cooperate to promote chromosomal instability and widely metastatic pancreatic ductal adenocarcinoma in mice. Cell Cancer 2005; 469-483.
Ladoire S, Arnould L, Apetoh L, et al. Pathologic complete response to neoadjuvant chemotherapy of breast carcinoma is associated with the disappearance of tumour-infiltrating foxp3+ regulatory T cells. Clin Cancer Res 2008;14:2413-2420.
Locher C, Conforti R, Aymeric L, et al. Desirable cell death during anticancer chemotherapy. Ann N Y Acad Sci 2010;1209:99-108.
Zitvogel L, Apetoh L, Ghiringhelli F, et al. The anticancer immune response: indispensable for therapeutic success? J Clin Invest 2008; 118:1991-2001.

## Claims

1. A whole cell *Mycobacterium* for use in the treatment of neoplastic disease in combination with an mTOR inhibitor, wherein the *Mycobacterium* is a non-pathogenic heat-killed *Mycobacterium.*

2. A *Mycobacterium* for use according to claim 1, wherein said *Mycobacterium* is selected from *M. vaccae, M.obuense, M. parafortuitum, M. aurum, M. indicus pranii, M. phlei* and combinations thereof.

3. A *Mycobacterium* for use according to claim 2, wherein the *Mycobacterium* is a rough variant.

4. A *Mycobacterium* for use according to any preceding claim, wherein said mTOR inhibitor is selected from sirolimus, everolimus, ridaforolimus, temsirolimus or metformin, and combinations thereof.

5. A *Mycobacterium* for use according to claim 4, wherein said mTOR inhibitor is rapamycin (sirolimus).

6. A *Mycobacterium* for use according to any preceding claim, wherein said *Mycobacterium* is administered to a subject before, concurrently with and/or after said mTOR inhibitor is administered.

7. A *Mycobacterium* for use according to any preceding claim, wherein said *Mycobacterium* and/or mTOR inhibitor is to be administered in repeat doses.

8. A *Mycobacterium* for use according to any preceding claim, wherein said neoplastic disease is uterine cancer, prostate cancer, liver cancer, renal cancer, lung cancer, breast cancer, colorectal cancer, pancreatic cancer, brain cancer, hepatocellular cancer, lymphoma, leukemia, gastric cancer, cervical cancer, ovarian cancer, thyroid cancer, melanoma, carcinoma, head and neck cancer, skin cancer or soft tissue sarcoma.

9. A *Mycobacterium* for use according to claim 8, wherein said neoplastic disease is pancreatic cancer.

10. A *Mycobacterium* for use according to any preceding claim, wherein said treatment reduces the size of a cancerous tumour such that it is operable and/or reduces the formation of metastases.

11. A *Mycobacterium* for use according to any preceding claim, wherein the *Mycobacterium* is present in a unit dose comprising an effective amount of non-pathogenic heat-killed *Mycobacterium* from 10⁷ to 10⁹ cells.

12. A *Mycobacterium* for use according to any preceding claim wherein the *Mycobacterium* is for administration via the parenteral, oral, sublingual, nasal or pulmonary route.

13. A *Mycobacterium* for use according to claim 12, wherein the parenteral route is selected from subcutaneous, intradermal, subdermal, intraperitonal, intravenous, or intravesicular injection.

14. A *Mycobacterium* for use according to claim 13, wherein the *Mycobacterium* is for administration via the intradermal route.

## Patentansprüche

1. Ganzzell-*Mycobacterium* zum Gebrauch beim Behandeln einer neoplastischen Erkrankung in Kombination mit einem mTOR-Hemmer, wobei das *Mycobacterium* ein nicht pathogenes durch Hitze abgetötetes *Mycobacterium* ist.

2. *Mycobacterium* zum Gebrauch nach Anspruch 1, wobei das *Mycobacterium* ausgewählt ist aus *M. vaccae, M. obuense, M. parafortuitum, M. aurum, M. indicus pranii, M. phlei* und Kombinationen daraus.

3. *Mycobacterium* zum Gebrauch nach Anspruch 2, wobei das *Mycobacterium* eine grobe Variante ist.

4. *Mycobacterium* zum Gebrauch nach einem der vorhergehenden Ansprüche, wobei der mTOR-Hemmer ausgewählt ist aus Sirolimus, Everolimus, Ridaforolimus, Temsirolimus und Metformin und Kombinationen daraus.

5. *Mycobacterium* zum Gebrauch nach Anspruch 4, wobei der mTOR-Hemmer Rapamycin (Sirolimus) ist.

6. *Mycobacterium* zum Gebrauch nach einem der vorhergehenden Ansprüche, wobei das *Mycobacterium* einem Subjekt vor, gleichzeitig mit und/oder nach dem Verabreichen des mTOR-Hemmers verabreicht wird.

7. *Mycobacterium* zum Gebrauch nach einem der vorhergehenden Ansprüche, wobei das *Mycobacterium* und/oder der mTOR-Hemmer in wiederholten Dosen zu verabreichen ist.

8. *Mycobacterium* zum Gebrauch nach einem der vorhergehenden Ansprüche, wobei es sich bei der neoplastischen Erkrankung um Gebärmutterkrebs, Prostatakrebs, Leberkrebs, Nierenkrebs, Lungenkrebs, Brustkrebs, Kolorektalkrebs, Bauchspeicheldrüsenkrebs, Hirnkrebs, Leberzellkrebs, Lymphom, Leukämie, Magenkrebs, Gebärmutterhalskrebs, Eierstockkrebs, Schilddrüsenkrebs, Melanom, Karzinom, Kopf-Hals-Krebs, Hautkrebs oder Weichteilsarkom handelt.

9. *Mycobacterium* zum Gebrauch nach Anspruch 8, wobei es sich bei der neoplastischen Erkrankung um Bauchspeicheldrüsenkrebs handelt.

10. *Mycobacterium* zum Gebrauch nach einem der vorhergehenden Ansprüche, wobei die Behandlung die Größe eines Krebsgeschwulstes derart reduziert, dass es operierbar ist, und/oder die Bildung von Metastasen reduziert.

11. *Mycobacterium* zum Gebrauch nach einem der vorhergehenden Ansprüche, wobei das *Mycobacterium* in einer eine wirksame Menge eines nicht pathogenen durch Hitze abgetöteten *Mycobacteriums* umfassenden Einheitsdosis von 10⁷ bis 10⁹ Zellen vorliegt.

12. *Mycobacterium* zum Gebrauch nach einem der vorhergehenden Ansprüche, wobei das *Mycobacterium* zum Verabreichen über den parenteralen, oralen, sublingualen, nasalen oder pulmonalen Weg vorgesehen ist.

13. *Mycobacterium* zum Gebrauch nach Anspruch 12, wobei der parenterale Weg ausgewählt ist aus subkutaner, intradermaler, subdermaler, intraperitonealer, intravenöser oder intravesikaler Injektion.

14. *Mycobacterium* zum Gebrauch nach Anspruch 13, wobei das *Mycobacterium* zum Verabreichen über den intradermalen Weg vorgesehen ist.

## Revendications

1. *Mycobacterium* à germes entiers pour une utilisation dans le traitement d'une maladie néoplasique en association avec un inhibiteur de mTOR, où le *Mycobacterium* est un *Mycobacterium* pathogène inactivé par la chaleur.

2. *Mycobacterium* pour une utilisation selon la revendication 1, où ledit *Mycobacterium* est choisi parmi *M. vaccae, M obuense, M. parafortuitum, M. aurum, M. indicus pranii, M. phlei* et les combinaisons de ceux-ci.

3. *Mycobacterium* pour une utilisation selon la revendication 2, où ledit *Mycobacterium* est une variante rugueuse.

4. *Mycobacterium* pour une utilisation selon l'une quelconque des revendications précédentes, où ledit inhibiteur de mTOR est choisi parmi le sirolimus, l'évérolimus, le ridaforolimus, le temsirolimus ou la metformine et les combinaisons de ceux-ci.

5. *Mycobacterium* pour une utilisation selon la revendication 4, où ledit inhibiteur de mTOR est la rapamycine (sirolimus).

6. *Mycobacterium* pour une utilisation selon l'une quelconque des revendications précédentes, où ledit *Mycobacterium* est administré à un sujet avant, pendant et/ou après l'administration dudit inhibiteur de mTOR.

7. *Mycobacterium* pour une utilisation selon l'une quelconque des revendications précédentes, où ledit *Mycobacterium* et/ou inhibiteur de mTOR doit être administré à doses répétées.

8. *Mycobacterium* pour une utilisation selon l'une quelconque des revendications précédentes, où ladite maladie néoplasique est le cancer de l'utérus, le cancer de la prostate, le cancer du foie, le cancer du rein, le cancer du poumon, le cancer du sein, le cancer colorectal, le cancer du pancréas, le cancer du cerveau, le cancer primitif du foie, le lymphome, la leucémie, le cancer de l'estomac, le cancer du col de l'utérus, le cancer de l'ovaire, le cancer de la thyroïde, le mélanome, le carcinome, le cancer de la tête et du cou, le cancer de la peau ou le sarcome des tissus mous.

9. *Mycobacterium* pour une utilisation selon la revendication 8, où ladite maladie néoplasique est le cancer du pancréas.

10. *Mycobacterium* pour une utilisation selon l'une quelconque des revendications précédentes, où ledit traitement réduit la taille d'une tumeur cancéreuse de telle sorte qu'elle est opérable et/ou réduit la formation de métastases.

11. *Mycobacterium* pour une utilisation selon l'une quelconque des revendications précédentes, où *Mycobacterium* est présent dans une dose unitaire comprenant une quantité efficace de *Mycobacterium* non pathogène inactivé par la chaleur à raison de 10⁷ à 10⁹ cellules.

12. *Mycobacterium* pour une utilisation selon l'une quelconque des revendications précédentes, où *Mycobacterium* est destiné à l'administration par voie parentérale, orale, sublinguale, nasale ou pulmonaire.

13. *Mycobacterium* pour une utilisation selon la revendication 12, où la voie parentérale est choisie parmi l'injection sous-cutanée, intradermique, subdermique, intrapéritonéale, intraveineuse ou intra-vésiculaire.

14. *Mycobacterium* pour une utilisation selon la revendication 13 où *Mycobacterium* est destiné à l'administration par voie intradermique.
